(19) Europäisches Patentamt
European Patent Office
Office européen des brevets

(11) **EP 3 217 144 A1**

(12) **EUROPÄISCHE PATENTANMELDUNG**

(43) Veröffentlichungstag:
**13.09.2017 Patentblatt 2017/37**

(51) Int Cl.:
**G01B 9/02** *(2006.01)* **G01B 11/24** *(2006.01)*
**A61B 3/10** *(2006.01)*

(21) Anmeldenummer: **16159900.6**

(22) Anmeldetag: **11.03.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **HAAG-STREIT AG**
**3098 Köniz (CH)**

(72) Erfinder:
• **Wagner, Jörg**
 **6370 Oberdorf (CH)**
• **Robledo, Lucio**
 **3013 Bern (CH)**
• **Cattin, Philippe**
 **5210 Windisch (CH)**

(74) Vertreter: **Gutmann, Samuel Oliver et al**
**Keller & Partner Patentanwälte AG**
**Eigerstrasse 2**
**Postfach**
**3000 Bern 14 (CH)**

(54) **AUGENVERMESSUNG**

(57) In einem Verfahren zum interferometrischen Erfassen von Messpunkten eines Bereich eines Auges werden mehrere Messpunkte mit einem Messstrahl entlang einer Trajektorie erfasst, wobei dieselbe Trajektorie vom Messstrahl mindestens in einem ersten Durchlauf und in einem zweiten Durchlauf im Bereich durchlaufen wird. Die Trajektorie des ersten Durchlaufs ist zur Trajektorie des zweiten Durchlaufs um einen Winkel rotiert und/oder um eine Strecke verschoben, um eine homogene Messpunktverteilung zu erreichen.

**Fig. 1**

EP 3 217 144 A1

## Beschreibung

Die Erfindung betrifft ein Verfahren zum interferometrischen Erfassen von Messpunkten eines Bereichs eines Auges, wobei mehrere Messpunkte mit einem Messstrahl entlang einer Trajektorie erfasst werden, wobei dieselbe Trajektorie vom Messstrahl mindestens in einem ersten Durchlauf und in einem zweiten Durchlauf im Bereich durchlaufen wird. Weiter betrifft die Erfindung eine Vorrichtung zur Durchführung des Verfahrens.

## Stand der Technik

Die vorliegende Erfindung betrifft das interferometrische Erfassen von Messpunkten auf einem Bereich eines Auges.

In der Augenheilkunde sind entsprechende Verfahren und Vorrichtungen bekannt. Typischerweise erfolgen solche Untersuchungen am Auge mit einem Interferometer. Als Interferometer werden zum Beispiel OCT (optische Kohärenztomographen) eingesetzt, welche das Auge an diskreten Positionen in lateraler Richtung abtasten und dabei entlang des optischen Strahls Streulichtprofile (axiale Profile) des Auges erfassen. Aufgrund der axialen Profile können drei dimensionale Messpunkte im Auge definiert werden welche bevorzugt eine oder mehrere optische Flächen repräsentieren.

Die Zeiss (US 9,101,294 B2) offenbart zum Beispiel ein Verfahren zur Verarbeitung von Daten aus einem OCT-Gerät, wobei mit unterschiedlichen Ansätzen eine Erhöhung der Präzision in der Berechnung von okularen Messungen erreicht werden soll. Die Methoden umfassen neue Scanmuster, die Verwendung von Techniken zur Bestimmung der transversalen Augenbewegung sowie ein verbesserter Algorithmus zur Augenbewegungskorrektur.

Die Methode umfasst insbesondere das Erfassen eines ersten kleinen Sets von Daten mittels OCT und die Verwendung der Daten zur Modellierung der Kornea. Das Modell wird anschliessend verwendet, um bei einer weiteren Messung mit einer dichteren Anzahl Messpunkte ein präziseres, bewegungs-korrigiertes Modell zu erstellen.

Weiter zeigt die Duke (US 8,403,481 B2) eine Methode zur Verminderung von Bewegungsartefakten bei OCT-Messungen. Im Verfahren werden mit einem Scanmuster Daten ermittelt, welche derart verteilt sind, dass zumindest gewisse räumlich adjazente Datenpunkte nicht sequentiell ermittelt wurden. Es kann dazu ein Scanmuster verwendet werden, bei welchem räumlich benachbarte Datenpunkte nichtsequentiell ermittelt werden. Zum Beispiel kann die Probe mit mehreren Serien Scanlinien gescannt werden, wobei einige der Scanlinien zwischen zuvor gemessenen Scanlinien liegen.

Als wesentliche Anforderungen an die Vermessung, insbesondere bei der Vermessung des Auges, werden einerseits eine geringe Gesamtdauer der Messung sowie eine hohe Auflösung, d.h. eine grosse Abdeckung der zu vermessenden Fläche, gestellt. Die geringe Gesamtdauer ist insbesondere aufgrund der nicht vermeidbaren Bewegungen des Auges während der Messung ein wesentliches Kriterium.

Die bekannten Verfahren haben den Nachteil, dass die Messungen relativ lange dauern. Aufgrund der relativ langen Messdauer besteht eine erhöhte Gefahr, dass die Messergebnisse durch Augenbewegungen des Patienten beeinflusst werden, womit lediglich ungenaue Messresultate erreicht werden können.

## Darstellung der Erfindung

Aufgabe der Erfindung ist es, ein dem eingangs genannten technischen Gebiet zugehörendes Verfahren zum interferometrischen Erfassen von Messpunkten auf einem Bereich eines Auges zu schaffen, welches eine besonders präzise Vermessung des Auges ermöglicht, wobei insbesondere Augenbewegungen besonders optimal korrigiert werden können.

Die Lösung der Aufgabe ist durch die Merkmale des Anspruchs 1 definiert. Gemäss der Erfindung ist die Trajektorie des ersten Durchlaufs zur Trajektorie des zweiten Durchlaufs um einen Winkel rotiert und/oder um eine Strecke verschoben, um eine homogenere Messpunktverteilung zu erreichen.

Die erfindungsgemässe Aufgabe wird ebenfalls durch eine Vorrichtung zur Durchführung eines solchen Verfahrens gelöst.

Dem **Messverfahren** liegt prinzipiell eine Abstandsmessung, insbesondere eine axiale Abstandsmessung in Z-Richtung (siehe unten), besonders bevorzugt ein axiales Streuprofil (ein sogenannter A-Scan) entlang der Strahlrichtung zugrunde. Bevorzugt erfolgt die Erfassung der Messpunkte mittels spectral domain OCT (SD-OCT) oder swept source OCT (SS-OCT), vorzugsweise in zeitlich konstanter Frequenz. Verfahren unter Verwendung von OCT haben sich in der Augenheilkunde etabliert, da diese bei streuenden Objekten wie einem Auge eingesetzt werden können und insbesondere eine relativ hohe Eindringtiefe bei gleichzeitiger hoher axialer Auflösung aufweisen.

Bei SD-OCT werden unterschiedliche optische Frequenzen eingesetzt. Das Licht wird dispergiert und mittels CCD oder CMOS-Sensor analysiert. Damit kann die volle Messtiefe mit einer einzigen Messung erreicht werden. Bei SS-OCT wird hingegen die optische Frequenz periodisch durchgestimmt und das Interferenzsignal zeitaufgelöst gemessen. Diese Techniken sind dem Fachmann hinreichend bekannt.

Das Erfassen von Abständen kann durch Peakdetektion im A-scan, Kantendetektion im A-scan oder durch Segmentierung von sequentiell erfassten A-scans erfolgen.

In Varianten können auch andere Techniken zur punktuellen Abstandsmessung oder zur axialen Profilmessung (Erfassung von A-Scans) eingesetzt werden (siehe Interferometer), zum Beispiel eine Laufzeitmessung mittels Laser oder dergleichen.

Nachfolgend werden **X, Y und Z-Koordinaten** als dreidimensionales Orthogonalsystem verwendet. Die Trajektorie wird vorzugsweise entlang einer X,Y-Ebene betrachtet und der Messstrahl weist zumindest in einer Messrichtung, insbesondere in Richtung eines Mittelpunkts des Bereichs, die Z-Richtung auf. Der Messstrahl kann aus dieser Lage entweder parallel verfahren, das heisst die Z-Richtung beibehalten, oder aber um einen Winkel in X-Richtung und einen zweiten Winkel in Y-Richtung verschwenkt werden. Weiterhin kann die Lageänderung auch durch eine Kombination dieser Varianten erreicht werden. Sofern nichts anderes erwähnt ist, wird ohne Beschränkung auf eine der Varianten, nachfolgend von der bevorzugten ersteren ausgegangen. Dazu können telezentrische Optiken vorgesehen sein, bei welchen ein Strahlversatz parallel erfolgen kann. Die Abstandsmessung bezieht sich auf die Z-Achse oder die Richtung des Messstrahls, wobei Änderungen des Brechungsindex entlang der Messachse zu Brechungseffekten führen können, die Richtungs- und Skalierungsänderungen des axialen Streuprofils nach sich ziehen können. Verfahren zur geometrischen Korrektur und Umrechnungen der gemessenen Längen sind dem Fachmann geläufig.

Die Trajektorie wird jeweils als **ebene Projektion** in einer X,Y-Ebene verstanden, welche das zu vermessende Auge schneidet oder einen geringen Abstand zum zu vermessenden Auge aufweist. In der Praxis kann die Trajektorie abhängig vom zu vermessenden Auge von dieser Beschreibung abweichen. Werden zum Beispiel mittels des Messstrahls entlang einer Trajektorie in regelmässigem Abstand Punkte erfasst, so liegen diese Punkte im Messbereich nur dann in konstantem Abstand, wenn der Messbereich eine zur ebenen Projektion parallele Ebene ist. Wird diese Trajektorie aber auf ein Auge projiziert, so liegen zeitlich benachbarte zentrumsnahe Messpunkte näher beieinander als zeitlich benachbarte Messpunkte im Randbereich des Auges.

Unter einem **axialen Längenprofil** oder A-Scan ist ein Profil des Auges entlang der z-Achse respektive der Strahlrichtung zu verstehen. Unter einem B-Scan wird ein axiales Profil entlang eines geraden Schnitts durch das Auge verstanden, welches sich aus einzelnen A-Scans zusammensetzt. Die axialen Längenprofile können zu einem dreidimensionalen Modell des Auges oder einem Schnitt durch das Auge zusammengefügt werden. Die Daten können jedoch auch anderweitig eingesetzt werden, zum Beispiel zur Simulation eines Strahlengangs, einer Korrekturlinse oder dergleichen.

Der **Bereich des Auges** ist nachfolgend nicht zwingend als Oberfläche der Hornhaut, sondern insbesondere auch als dreidimensionaler Bereich zu verstehen. So können mit Hilfe von OCT-Verfahren Tiefenprofile des Auges erstellt werden, welche nachfolgend gleichermassen unter den Begriff "Bereich" fallen.

Der **Abdeckungsgrad** ist derjenige Anteil der zu vermessenden Fläche, in welchem der Abstand von jedem beliebigen Punkt zum nächstgelegenen Messpunkt einen kritischen Wert nicht überschreitet. Für Messungen der Topographie des Auges wird üblicherweise ein Abdeckungsgrad von 100 % gefordert, wobei die zu vermessende Fläche mindestens einen Durchmesser von 7.5 mm aufweisen und der kritische Abstand 0.5 mm betragen sollte. Je nach Anforderung, zum Beispiel bei der Realisierung kürzerer Messzeiten, können jedoch auch geringere Abdeckungsgrade oder kleinere Messflächen verwendet werden. Die Flächen und Abstände beziehen sich auf die X,Y-Ebene in welcher die Trajektorie definiert wird.

Unter der **homogeneren Messpunktverteilung** wird ein vergrösserter Abdeckungsgrad verstanden im Vergleich zu einem zweiten Durchlauf ohne Rotation oder Verschieben der Trajektorie. Die homogenere Messpunktverteilung wird damit insbesondere dann erreicht, wenn mindestens ein Messpunkt des zweiten Durchlaufs nicht auf einem Messpunkt des ersten Durchlaufs zu liegen kommt.

Die **Trajektorie** ist nachfolgend in Bezug auf die X,Y-Ebene als zweidimensionale Kurve definiert, sofern nichts anderes erwähnt ist. Dem Fachmann ist jedoch klar, dass die Trajektorie in der Anwendung als Projektion auf einen Körper typischerweise eine dreidimensionale Raumkurve darstellt, welche gegenüber der Trajektorie verzerrt ist. Da diese Raumkurve aber sowohl von der Trajektorienform als auch von der Körperform abhängt und damit in grossen Bereichen variieren kann, wird nicht weiter darauf eingegangen. Die Trajektorie muss indes nicht zwingend mathematisch exakt einer Funktion oder Definition folgen. Vorzugsweise weist die Trajektorie eine Form auf, welche zumindest für die einzelnen Messpunkte auf der respektive einer der obig beschriebenen Trajektorie liegt. In diesem Fall werden die Funktionen respektive die Formen schliesslich erst durch eine Interpolation der Messpunkte definiert. In der Praxis können die Messpunkte aber auch geringfügig von der Trajektorie abweichen. Die mittlere Abweichung von der Trajektorie kann je nach Anzahl Messpunkte und Grösse des Objekts zum Beispiel weniger als 5 %, vorzugsweise weniger als 1 % des Durchmessers des Umkreises der zu vermessenden Fläche betragen.

Unter einem **Durchlauf** wird nachfolgend das Erfassen von Messpunkten auf der Trajektorie verstanden, wobei die Trajektorie genau einmal vollständig durchlaufen wird. Statt des Begriffs "Durchlauf" wird auch der gleichwertige Begriff "Zyklus" verwendet.

Die **Rotation und/oder Verschiebung der Trajektorie** ist dahingehend zu verstehen, dass zumindest ein Punkt der Trajektorie des ersten Durchlaufs nicht mit der Trajektorie des zweiten Durchlaufs übereinstimmt. Vorzugsweise stimmt mindestens ein Messpunkt auf der Trajektorie des ersten Durchlaufs nicht mit einem Messpunkt auf der Trajektorie des zweiten Durchlaufs überein. Besonders bevorzugt weist die Trajektorie des ersten Durchlaufs mit der Trajektorie des zweiten Durchlaufs ausschliesslich Schnittpunkte auf, so dass keine zwei benachbarten Messpunkte von der Trajektorie des ersten Durchlaufs und der Trajektorie des zweiten Durchlaufs erfasst sind. Die Rotation und/oder Verschiebung der Trajektorie ist als Relativbewegung zwischen der Trajektorie und dem Auge zu verstehen.

Die Rotation und/oder Verschiebung der Trajektorie ist weiter dahingehend zu interpretieren, dass eine der folgenden Optionen vorliegt:

- Ausschliesslich eine Rotation der Trajektorie; oder
- ausschliesslich eine Verschiebung der Trajektorie; oder
- eine Kombination zwischen einer Rotation und einer Verschiebung der Trajektorie.

Unter der **Rotation der Trajektorie** wird eine Drehung der Trajektorie um die zur Z-Achse parallelen Rotationsachse verstanden, wobei die Rotationsachse vorzugsweise durch einen Mittelpunkt der Trajektorie verläuft. Der Mittelpunkt der Trajektorie kann dabei auf verschiedene Arten definiert sein. Zum Beispiel kann der Mittelpunkt als Anfangs- oder Endpunkt der Trajektorie in Bezug auf einen Durchlauf definiert sein. Anderseits kann der Mittelpunkt der Trajektorie auch als Mittelpunkt eines Umkreises der Trajektorie definiert sein. Besonders bevorzugt liegt der Mittelpunkt ungefähr auf dem Apex, einem Symmetrievektor oder einer optischen Achse des Auges
Unter der **Verschiebung** der Trajektorie wird ein laterales Verschieben der Trajektorie in der X,Y-Ebene verstanden.

Erfindungsgemäss ist nun die Trajektorie des ersten Durchlaufs zur Trajektorie des zweiten Durchlaufs um einen Winkel rotiert und/oder um eine Strecke verschoben, so dass eine homogenere Messpunktverteilung erreicht ist. Die homogenere Messpunktverteilung wird dadurch erreicht, dass durch die Rotation respektive die Verschiebung der Trajektorie zumindest ein Messpunkt des zweiten Durchlaufs nicht auf einem Messpunkt des ersten Durchlaufs liegt. Besonders bevorzugt weisen die beiden Trajektorien des ersten respektive des zweiten Durchlaufs keine gemeinsamen benachbarten Messpunkte auf, so dass die Trajektorien ausschliesslich in Form von Schnittpunkten aufeinander treffen. Damit wird durch die Rotation respektive die Verschiebung eine Streuung der Messpunkte weiter vergrössert.

Da erfindungsgemäss genau eine Trajektorie eingesetzt werden kann um eine homogenere Messpunktverteilung zu erreichen, wird ein besonders einfaches Verfahren zur Erfassung von Messpunkten auf einem Auge geschaffen, welches zudem auch in der Auswertung der Messdaten besonders vorteilhaft ist.

Vorzugsweise schneidet jede innerhalb der Trajektorie verlaufende Gerade die Trajektorie in mindestens zwei zueinander beabstandeten Punkten. Damit wird eine Trajektorie erhalten, welche bereits für sich eine gute Messpunktverteilung aufweist.

Alternativ können auch Trajektorien eingesetzt werden, welche von einer solchen Geraden lediglich in einem Punkt geschnitten werden.

In einer besonders vorteilhaften Ausführungsform des Verfahrens ist die Trajektorie derart ausgebildet, dass:

- im Falle einer Rotation der Trajektorie, die Messpunkteabstände radial, insbesondere zum Rotationspunkt der Trajektorie, besonders optimal verteilt sind;
- Im Falle einer Verschiebung der Trajektorie die Messpunkte insbesondere in einer die Verschiebungsrichtung kreuzenden Richtung besonders optimal verteilt sind.

Bei einer kombinierten Bewegung der Trajektorie durch Rotation und Verschiebung kann die Messpunktverteilung der Trajektorie derart gewählt werden, dass die jeweiligen Beträge der Rotation respektive der Verschiebung entsprechend berücksichtig sind. Sofern zum Beispiel der Rotationsanteil gross ist, kann eine homogene Messpunktverteilung in radialer Richtung mehr gewichtet werden und vice versa.

In Varianten kann aber die Messpunktverteilung auf der Trajektorie auch weitgehend ausser Acht gelassen werden.

Vorzugsweise stimmt ein Anfangspunkt der zweiten Trajektorie mit dem Endpunkt der ersten Trajektorie überein. Damit wird erreicht, dass das Messverfahren kontinuierlich, das heisst ohne Sprünge, durchgeführt werden kann. Mit dem Interferometer kann damit kontinuierlich, zum Beispiel in zeitlich konstantem Abstand, gemessen werden. Dies kann zum Beispiel dadurch erreicht werden, dass als Anfangspunkt und Endpunkt jeweils derselbe Punkt gewählt wird. Dieser kann zum Beispiel im Mittelpunkt des Bereichs liegen.

In Varianten kann der Anfangspunkt der zweiten Trajektorie auch nicht mit dem Endpunkt der ersten Trajektorie übereinstimmen.

Bevorzugt deckt die Trajektorie den Bereich ab. Unter dem Begriff "Abdecken" wird verstanden, dass der Bereich eine Teilmenge des Umkreisinhalts um die Trajektorie ist. Damit wird erreicht, dass bereits beim ersten Durchlauf der Trajektorie das Auge in diesem Bereich im Wesentlichen, wenn auch unter Umständen noch mit relativ geringer Messpunktdichte, vermessen werden kann. Dies ist insbesondere dann von Vorteil, wenn bereits nach dem ersten Durchlauf gewisse Rückschlüsse gezogen werden sollen, insbesondere zum Beispiel das Auge relativ zum Messgerät lokalisiert werden soll.

In Varianten kann die Trajektorie auch so gestaltet sein, dass sie den Bereich nicht abdeckt. In diesem Fall kann die Trajektorie zum Beispiel derart geschaffen sein, dass der Bereich bei geeigneter Rotation und/oder Verschiebung nach dem zweiten Durchlauf abgedeckt ist. Die Trajektorie kann aber auch derart gestaltet sein, dass die Abdeckung erst nach mehr als zwei Durchläufen erreicht ist, zum Beispiel nach mehr als 3, 5, 10 Durchläufen.

Vorzugsweise wird die Trajektorie kontinuierlich um einen Winkel rotiert und/oder um eine Strecke verschoben. Die kontinuierliche Bewegung der Trajektorie, das heisst die kontinuierliche Rotation und/oder Verschiebung, hat den Vorteil, dass das Messverfahren während der Bewegung der Trajektorie nicht unterbrochen werden muss. Das Messverfahren kann damit ohne Unterbruch durchgeführt werden, womit eine besonders schnelle und effiziente Vermessung eines Auges ermöglicht wird. Die Geschwindigkeit der Vermessung ist dabei von grossem Interesse, da damit Bewegungsartefakte des Auges während der Messung minimiert werden können. Weiter kann damit vermieden werden, dass beim Übergang zwischen den Durchläufen eine Unstetigkeit oder ein verkleinerter Bewegungsradius entsteht, welche die Messgeschwindigkeit negativ beeinflussen kann. Durch die kontinuierliche respektive gleitende Bewegung der Trajektorie während des Messvorgangs kann somit weitgehend ein sich für die Messgeschwindigkeit negativ auswirkender verringerter Kurvenradius vermieden werden.

In Varianten kann die Rotation und/oder die Verschiebung der Trajektorie auch jeweils zwischen den Durchläufen erfolgen. In diesem Fall ist es von besonderem Vorteil, wenn der Anfangspunkt des zweiten Durchlaufs und der Endpunkt des ersten Durchlaufs übereinstimmen und zum Beispiel im Mittelpunkt der Trajektorie oder des Bereichs liegen.

In einer bevorzugten Ausführungsform ist die Trajektorie des zweiten Durchlaufs zur Trajektorie des ersten Durchlaufs ausschliesslich aufgrund einer Bewegung des Auges um einen Winkel rotiert und/oder um eine Strecke verschoben, womit eine homogenere Messpunktverteilung erreicht ist. Damit wird ein besonders einfaches Messverfahren erreicht, da nicht die Trajektorie, sondern das Auge bewegt ist. Die Bewegung des Auges ist dabei vorzugsweise die typischerweise nicht verhinderbare Bewegung des Auges des Probanden während der Messung. Obschon der Proband während der Messung zum Beispiel das Kinn aufstützt und die Stirn gegen eine weitere Stütze presst, sind minimale Bewegungen des Auges nicht zu vermeiden. Bei diesen Bewegungen wird zwischen axialen Bewegungen (in der Z-Achse) und lateraler Bewegung (in der X,Y-Ebene) unterschieden. Die axiale Bewegung der Cornea liegt typischerweise in einem Bereich von 100-200 $\mu$m. Die axiale Bewegung ist in der Regel eher langsam und wird im Wesentlichen durch den Puls ausgelöst. Die laterale Bewegung der Cornea liegt ebenfalls typischerweise im Bereich von 100-200 $\mu$m. Die laterale Bewegung besteht etwa zu gleichen Teilen aus kleinsten Rotationen des Auges (langsamer Drift, schnelle Mikrosakkaden etc.) und aus lateralen Kopfbewegungen. Die lateralen Kopfbewegungen sind eher langsamer als die Rotationen, weisen aber ähnliche Amplitude auf. Diese Angaben beziehen sich auf die von einem idealen Probanden nicht beinflussbaren, natürlichen Bewegungen, das heisst, die obigen Angaben liegen eher im unteren Bereich der zu erwartenden Bewegungen. In der Praxis können die Bewegungen auch grösser ausfallen. In einem besonders bevorzugten Verfahren wird die Trajektorie ausschliesslich aufgrund der natürlichen Augenbewegung rotiert und/oder verschoben. In einer weiteren Ausführungsform kann das Auge auch aktiv bewegt werden, z.B. indem die Halterung des Kopfes des Probanden bewegt wird oder indem ein Fixationslicht oder dergleichen für das Auge des Probanden bewegt wird. Damit kann die Bewegung des Auges auch aktiv beeinflusst und gegebenenfalls eine homogenere Messpunktverteilung erreicht werden.

In besonders bevorzugten Variante werden die Bewegungen aktiv durch das Messgerät erreicht, wobei der Messstrahl derart gesteuert wird, dass die Trajektorie des zweiten Durchlaufs gegenüber der Trajektorie des ersten Durchlaufs um einen Winkel rotiert und/oder um eine Strecke verschoben ist.

Vorzugsweise ist die Trajektorie nach jedem Durchlauf um einen Winkel von m*360°/n, mit m, n $\geq$ 2 und m $\neq$ n rotiert. Dabei sind m und n natürliche Zahlen und m ist vorzugsweise grösser 0. Der Quotient m/n ist weiter trivialerweise zwischen 0 und 1 zu wählen. Vorzugsweise wird der Winkel derart gewählt und ist der Trajektorie derart angepasst, dass bei jedem Durchlauf Messpunkte erfasst werden, welche bei dem vorhergehenden Durchlauf oder den vorhergegangenen Durchläufen nicht erfasst wurden. Damit wird eine besonders optimale Messpunktdichte erreicht, womit wiederum eine besonders präzise Vermessung des Auges erreicht wird. Besonders bevorzugt ist der Quotient m/n grösser als 0.01, weiter bevorzugt grösser als 0.1, besonders bevorzugt grösser als 0.2. Damit wird erreicht, dass durch die rotierten Trajektorien bereits nach möglichst wenigen Durchläufen eine weitgehend regelmässige Messpunktverteilung erreicht ist. Bei einem Quotienten von m/n=0.2 würde die weitgehend regelmässige Messpunktverteilung zum Beispiel nach 5 Durchläufen erreicht. Bei einem Quotienten von m/n=0.375 bereit nach ungefähr 3 Durchläufen. In einer weiteren vorteilhaften Ausgestaltung ist m/n derart gewählt, dass der k*m/n, wobei k eine natürliche Zahl ist, erst für grosse k ganzzahlig wird. Damit wird weiter erreicht, dass einerseits mit einer geringen Anzahl Durchläufen bereits eine weitgehend regelmässige Messpunktverteilung erreicht werden kann, wobei aber erst nach einer grossen Anzahl an Durchläufen die Identität der Trajektorie erreicht ist. Im Beispiel mit m/n=0.375 wird bereits nach ungefähr 3 Durchläufen eine weitgehend regelmässige Messpunktverteilung erreicht, während die Identität erst nach 8 Durchläufen erreicht ist. Damit sind m und n in einem bevorzugten Verfahren teilerfremd, das heisst ggT(m, n)=1, womit die Identität nach n Durchläufen erreicht ist, während bereits nach $\left\lceil \frac{n}{m} \right\rceil$ (n/m aufgerundet) Durchläufen eine weitgehend regelmässige Messpunktverteilung erreicht ist. Der Wert m/n kann weiter beispielsweise 4/9, 5/9, 7/15, 7/16, 49/128 etc. betragen.

In Varianten kann auf die Rotation der Trajektorie auch verzichtet werden. In diesem Fall kann die Trajektorie zum Beispiel ausschliesslich verschoben werden.

Bevorzugt ist die Trajektorie nach dem zweiten Durchlauf im Vergleich zum ersten Durchlauf um einen Winkel zwischen $360 * 0.9 * \left(\frac{3\sqrt{5}}{2}\right)$ und $360 * 1.1 * \left(\frac{3\sqrt{5}}{2}\right)$, vorzugsweise zwischen $360 * 0.95 * \left(\frac{3\sqrt{5}}{2}\right)$ und $360 * 1.05 * \left(\frac{3\sqrt{5}}{2}\right)$, besonders bevorzugt zwischen $360 * 0.99 * \left(\frac{3\sqrt{5}}{2}\right)$ und $360 * 1.01 * \left(\frac{3\sqrt{5}}{2}\right)$, insbesondere um einen Winkel von ungefähr $360 * \left(\frac{3\sqrt{5}}{2}\right)$ rotiert. Der Betrag von $360 * \left(\frac{3\sqrt{5}}{2}\right)$ entspricht dabei dem goldenen Winkel. Im Idealfall, das heisst bei der Verwendung des goldenen Winkels, wird erreicht, dass kein Durchlauf mit einem vorhergehenden Durchlauf deckungsgleich ist. Damit wird insbesondere erreicht, dass die Messpunktverteilung mit jedem Durchlauf vergrössert wird. Der goldene Winkel hat aber weiter den Vorteil, dass die Zunahme der Messpunktverteilung im Wesentlichen gleichmässig erfolgt. Damit kann das Messverfahren nach einer beliebigen Anzahl Durchläufen unterbrochen werden, ohne dass eine übermässig asymmetrische Messpunktverteilung riskiert wird. Dadurch können auch Abschnitte oder ganze Durchläufe entfernt werden welche als Ausreisser identifiziert wurden. Zur Ausreisserrekennung werden bevorzugt die einzelnen Durchläufe zur Modellierung des Bereichs verwendet. Dies ist insbesondere möglich, wenn dabei der Abdeckungsgrad erhalten bleibt. Würde zum Beispiel ein sehr kleiner Winkel, wie zum Beispiel 3.6° gewählt, so würde bei einer zweischleifigen Trajektorie nach zum Beispiel 10 Durchläufen eine übermässig asymmetrische Messpunktverteilung erreicht.

In Varianten können aber auch andere Winkel vorgesehen sein, z.B. 0.375*360°, so dass zum Beispiel nach 8 Durchläufen die Identität erreicht ist oder 0.4375*360°, so dass nach 16 Durchläufen die Identität erreicht ist etc. (siehe oben).

Vorzugsweise wird mindestens anhand der Messpunkte des ersten Durchlaufs ein erstes Modell des Bereichs und anhand der Messpunkte des zweiten Durchlaufs ein zweites Modell des Bereichs berechnet. Dabei handelt es sich vorzugsweise um geometrische Modelle. Die Berechnung des Models kann sowohl die Berechnung der Hornhautoberfläche wie auch weiterer Ebenen, wie die Hornhautrückfläche, die Linse etc. umfassen. Mit Hilfe dieser, bevorzugt geometrischen Modelle kann besonders einfach die Position des Auges ermittelt werden. Aus den Modellen können dann jeweils die Position und gegebenenfalls die Ausrichtung der Hornhaut bestimmt werden. Bevorzugt erfolgt die Modellierung mittels Zernike Polynomen.

In Varianten kann auf die Berechnung des Modells verzichtet werden.

Im Unterschied zur traditionellen Fotografie, mit welcher zwei- oder dreidimensionale Momentaufnahmen erreicht werden können, basiert die Vermessung mittels OCT auf der sequentiellen Aufnahme von eindimensionalen A-Scans. Damit wird die Messdauer erhöht, so dass die Aufnahmen anfällig für Augenbewegungen, Lidschläge und dergleichen werden. Damit besteht die Gefahr, dass die Scans fehlerhaft oder unvollständig sein können. Daher besteht bei OCT-Messungen die Motivation, Bewegungsartefakte eliminieren zu können.

Daher wird bevorzugt anhand des mindestens ersten Modells und des zweiten Modells eine Raumkurve berechnet, welche die Bewegung des Auges repräsentiert. Die daraus interpolierte Bewegungstrajektorie lässt sich dann auf die gemessenen Punkte respektive auf die Modelle anwenden, um eine genauere Modellierung zu erreichen. Es ist dabei von besonderem Vorteil, wenn die einzelnen Modelle, welche typischerweise jeweils auf Basis eines Durchlaufs oder eines Teils eines Durchlaufs ermittelt werden, zur Bestimmung der Bewegung gleichberechtigt sind.

In Varianten kann auf eine Berechnung der Raumkurve zur Repräsentierung der Augenbewegung auch verzichtet werden.

Vorzugsweise werden für das erste Modell und das zweite Modell Symmetrievektoren bestimmt, anhand derer die Raumkurve berechnet wird, welche die Bewegung des Auges repräsentiert. Unter dem Symmetrievektor wird ein Vektor verstanden, um dessen Achse das Auge im Wesentlichen symmetrisch ist. Typischerweise liegt dieser Symmetrievektor ungefähr im Bereich einer optischen Achse des Auges. Der Symmetrievektor kann über eine oder mehrere Ebenen des Auges, wie zum Beispiel Hornhautvorderseite, Hornhauthinterseite, Linsenoberflächen etc., bestimmt werden.

In Varianten kann auf die Bestimmung von Symmetrievektoren verzichtet werden. Zur Ausrichtung des Auges kann auch eine Asymmetrie des Auges identifiziert werden, welche als Referenz für die Position und Ausrichtung des Auges dienen kann.

Vorzugsweise umfasst die Bewegung translatorische und rotierende Bewegungsanteile. Der Symmetrievektor kann damit einerseits die Ausrichtung der Symmetrieachse des Auges sowie eine Rotation des Auges erfassen. Der Symmetrievektor kann damit einerseits die Ausrichtung der Symmetrieachse des Auges sowie eine Rotation des Auges erfassen. Gesamthaft wird damit weiter das Verfahren zur Vermessung eines Bereichs eines Auges verbessert, da anhand dieser Daten eine Bewegungsrekonstruktion des Auges besonders präzise berechnet werden kann.

In Varianten kann zum Beispiel auf den rotierenden Bewegungsanteil verzichtet werden.

Vorzugsweise wird aus den Modellen ein mittleres Modell berechnet. Mit diesem Verfahren kann der Bereich des Auges besonders präzise modelliert werden, insbesondere da die jeweiligen Modelle vorzugsweise paarweise auf mindestens zum Teil nicht gemeinsamen Messpunkten basieren. Im Verfahren werden mehrere Durchläufe gemessen. Anhand jedes einzelnen Durchlaufs wird ein Modell, zum Beispiel eine Modell der Hornhaut, berechnet. Weiter wird für jedes Modell eine Position bestimmt, welche zum Beispiel mittels Symmetrievektor ermittelt werden kann (siehe oben). Anhand der Positionen kann eine Bewegungstrajektorie des Auges ermittelt werden. Im Anschluss werden die Positionen der einzelnen Modelle anhand der Bewegungstrajektorie ausgerichtet. Anhand der ausgerichteten Modelle wird schliesslich ein mittleres Modell berechnet.

In Varianten können auch die einzelnen Messpunkte der Durchläufe ausgerichtet werden, welche im Anschluss zu einem Modell verrechnet werden.

In einem weiteren bevorzugten Verfahren werden statt der Modelle die Messpunkte selbst aufgrund des ersten Modells und des zweiten Modells zur Reduktion von Bewegungsartefakten zu korrigierten Messpunkten korrigiert.

Vorzugsweise wird zur Korrektur der Messpunkte die Bewegungstrajektorie anhand der Positionen und Orientierungen der Modelle so interpoliert, dass sich für jeden Messpunkt eine interpolierte Position und Ausrichtung des Auges ergibt.

In Varianten kann auf die Interpolation auch verzichtet werden.

Bevorzugt werden die korrigierten Messpunkte des ersten Durchlaufs und des zweiten Durchlaufs zu einem kumulierten Modell zusammengefasst. Das kumulierte Modell umfasst damit die um die Bewegungsartefakte bereinigten Messpunkte mehrerer Durchläufe, womit eine besonders hohe Messpunktdichte und damit eine besonders hohe Präzision bei der Vermessung des Bereichs des Auges erreicht wird.

In Varianten kann auf die Berechnung des kumulierten Modells auf Basis der einzelnen bewegungskorrigierten Messpunkte auch verzichtet werden. In dem Fall können zum Beispiel wie oben erläutert die einzelnen korrigierten Modelle zu einem mittleren Modell verrechnet werden.

Vorzugswiese wird die Trajektorie in m Durchläufen durchlaufen, wobei m derart gewählt ist, dass ein mittlerer Messpunktabstand kleiner als eine vorherbestimmte erwartete laterale Bewegung des Auges ist. In einer besonders bevorzugten Ausführungsform ist m derart gewählt, dass der mittlere Messpunktabstand ungefähr mit der vorherbestimmten erwarteten lateralen Bewegung des Auges übereinstimmt. Ab dieser Anzahl an Durchläufen ist die Platzierung der neuen Messpunkte zwischen den bereits gemessenen Messpunkten aufgrund der Augenbewegung zufällig, womit unter Umständen auf die Verschiebung und/oder Rotation der Trajektorie sogar verzichtet werden kann.

In Varianten kann m auch kleiner gewählt sein. In diesem Fall könnte das Messverfahren schneller aber mit geringerer Messpunktdichte durchgeführt werden.

In der Vermessung der Hornhaut mit OCT wird typischerweise der zentrale Bereich der Kornea an diskreten Positionen gescannt. Anhand der Messpunkte können Topografiekarten des Bereichs erstellt werden. Für die Rekonstruktion der Oberfläche werden häufig Zernike-Polynome eingesetzt. Während bei einer normalen Kornea ein Zernike-Polynom 4ter Ordnung ausreicht, kann für ein abnormales Auge die 7te oder höhere Ordnung notwendig sein. Da zudem die Zernike-Polynome im diskreten Fall ihre Orthogonalität verlieren, hat das verwendete Scanmuster einen direkten Einfluss auf die numerische Stabilität der Rekonstruktion und limitiert die Ordnung der Zernike-Rekonstruktion.

Vorzugsweise weist die Trajektorie daher die Form einer Spirale, insbesondere einer Fermatspirale; einer Hypotrochoiden; eines Rasters, insbesondere parallele Linien; oder von radial angeordneter Schleifen mit einem gemeinsamen Schnittpunkt auf. Diese Formen der Trajektorie haben sich im Zusammenhang mit dem erfindungsgemässen Verfahren als besonders vorteilhaft herausgestellt. Die rasterförmigen Trajektorien sind zwar besonders einfach zum Scannen, da lediglich gerade Linien abgefahren werden müssen. Diese haben aber den Nachteil, dass relativ kleine Radien durchlaufen werden müssen, womit die Geschwindigkeit in diesen Bereichen typischerweise reduziert ist. Damit wird der Scanvorgang verlangsamt, womit wiederum Bewegungsartefakte verstärkt auftreten können. Es hat sich daher als vorteilhaft herausgestellt, meridionale Trajektorien zu verwenden, welche um einen gemeinsamen Punkt, insbesondere den Apex des Auges, angeordnet sind. Ein Vorteil dieses Verfahrens ist weiter die vereinfachte Bewegungskorrektur, da sämtliche Meridionale den Apex des Auges durchfahren. Anderseits bestehen die Nachteile darin, dass in der Regel keine optimale Messpunktverteilung erreichbar ist, womit wiederum die Zernike-Rekonstruktion beeinträchtigt wird und dass typischerweise geringe Radien durchlaufen werden müssen.

Besonders bevorzugt handelt es sich deshalb bei der Form der Trajektorie um eine Fermatspirale oder um eine Hypotrochoide. Diese Formen weisen besonders günstige Messpunktverteilungen auf, wobei bei einer geeigneten Rotation der Trajektorie die Messpunktdichte besonders optimal erhöht und eine stabilere Zernike-Rekonstruktion erreicht werden kann.

Besonders bevorzugt handelt es sich bei der Trajektorie um eine Fermatspirale der Form:

$$r = \pm\theta^{1/2}$$

Wobei r der Radius und θ der Winkel der Spiralpunkte in Polar Koordinaten sind. In einem besonders bevorzugten Verfahren ist die Trajektorie derart angepasst, dass der Radius linear von Null bis zum Radius des Scanmusters und zurück fährt, womit ein Durchlauf definiert ist. Damit wird eine Spirale erreicht, welche nach aussen, nach innen etc. verfährt.

In Varianten können aber auch andere Trajektorien, insbesondere andere Spiralen etc., vorgesehen sein.

In einer weiteren vorteilhaften Ausführungsform wird in jedem Durchlauf die Trajektorie kontinuierlich um einen Winkel rotiert. Wird dazu der goldene Winkel gewählt, so wird der Bereich mit kontinuierlich vergrösserter Messpunktdichte abgedeckt, das heisst, mit jedem Durchlauf wird der mittlere Abstand zwischen den Messpunkten verkleinert (siehe oben).

Dem Fachmann sind jedoch beliebige andere Trajektorien bekannt, welche im vorliegenden Verfahren eingesetzt werden können.

Weiter ist dem Fachmann auch klar, dass die Trajektorie generell nicht auf die exakte Einhaltung der durch die Formeln gebildeten Graphen beschränkt ist. Eine Trajektorie oder ein Scanmuster kann auch von der mathematisch exakten Form abweichen. So kann zum Beispiel die mit dem Messstrahl ermittelte Punkteschar lediglich als Interpolation näherungsweise einer solchen Funktion entsprechen.

Aus der nachfolgenden Detailbeschreibung und der Gesamtheit der Patentansprüche ergeben sich weitere vorteilhafte Ausführungsformen und Merkmalskombinationen der Erfindung.

**Kurze Beschreibung der Zeichnungen**

Die zur Erläuterung des Ausführungsbeispiels verwendeten Zeichnungen zeigen:

Fig. 1    einen ersten Zyklus der Fermatspirale;

Fig. 2    einen kompletten Scan mit der Fermatspirale, mit acht Zyklen;

Fig. 3    eine zweite Ausführungsform einer möglichen Trajektorie;

Fig. 4    eine dritte Ausführungsform einer möglichen Trajektorie.

Grundsätzlich sind in den Figuren gleiche Teile mit gleichen Bezugszeichen versehen.

**Wege zur Ausführung der Erfindung**

In einer ersten bevorzugten Ausführungsform weist die Trajektorie die Form einer Fermatspirale auf und ist wie folgt definiert:

$$r = \pm\theta^{1/2}$$

Wobei r der Radius und θ der Winkel der Spiralpunkte in Polar Koordinaten sind. Die Spirale kann um folgende Parameter und Eigenschaften erweitert sein:

R: maximaler Radius, ab welchem die Spirale wieder zum Mittelpunkt läuft.

M: Anzahl Umdrehungen für einen Sweep (bei den nachfolgenden Formeln im Index mit "s" gekennzeichnet). Diese Zahl definiert, wie oft die Spirale um den Mittelpunkt läuft, bis der maximale Radius erreicht ist.

$\theta_G$: Winkel, um welchen das Pattern während eines Durchlaufs gedreht wird

Mit Sweep wird die Trajektorie vom Mittelpunkt bis zum Umkehrpunkt am Rand und vice versa bezeichnet. Mit Zyklus wird ein Sweep (im Index jeweils mit s abgekürzt) nach aussen und anschliessendem Sweep nach innen bezeichnet. Der Begriff "Durchlauf" ist gleichbedeutend mit dem Begriff "Zyklus".

Daraus ergeben sich folgende Hilfsparameter zur Definition der Trajektorie:

$$\theta_s = M * 2\pi - \frac{\pi}{2} + \frac{\theta_G}{2}$$

$$a = \left(\frac{R^2}{\theta_s}\right)^{1/2}$$

Somit ergibt sich für die Fermatspirale:

$$r = \begin{cases} a(\theta \bmod \theta_s)^{1/2} & f\ddot{u}r\ \theta \bmod 2\theta_s \leq \theta_s\ und\ \theta \bmod 4\theta_s \leq 2\theta_s \\ a\big(\theta_s - (\theta \bmod \theta_s)\big)^{1/2} & f\ddot{u}r\ \theta \bmod 2\theta_s > \theta_s\ und\ \theta \bmod 4\theta_s \leq 2\theta_s \\ -a(\theta \bmod \theta_s)^{1/2} & f\ddot{u}r\ \theta \bmod 2\theta_s \leq \theta_s\ und\ \theta \bmod 4\theta_s > 2\theta_s \\ -a\big(\theta_s - (\theta \bmod \theta_s)\big)^{1/2} & f\ddot{u}r\ \theta \bmod 2\theta_s > \theta_s\ und\ \theta \bmod 4\theta_s > 2\theta_s \end{cases}$$

Das Abtastmuster ergibt sich nun aus der zeitlichen Folge von Messpunkten respektive Abtastpunkten auf der Trajektorie. Eine gleichmässige Verteilung der Punkte in der Fläche kann dadurch erreicht werden, dass die Abtastpunkte regelmässig in θ verteilt sind. Mit $N_{zyklus}$ für die Anzahl Punkte pro Zyklus und N für die Anzahl Messpunkte ergibt sich für θ:

$$\theta = \frac{n * 2 * \theta_s}{N_{Zyklus}} \quad f\ddot{u}r\ n = 0, 1, 2, \dots, N$$

Im Idealfall mit theoretisch unendlicher Erhöhung der Messpunktdichte, wird für θ der goldene Winkel eingesetzt (siehe oben). In der Praxis ist es jedoch üblicherweise ausreichend, wenn sich die Trajektorie nach einer endlichen Anzahl Sweeps oder Zyklen respektive Durchläufen wiederholt. Dazu kann statt des goldenen Winkels zum Beispiel $\theta_G$ = 0.375 * 2π, M = 8; $N_{Zyklus}$ = 4096; N = 8 * 4096 = 32768 gewählt sein. Bei einer A-Scan-Rate von 10 kHz würde eine Messung zum Beispiel 3.28 Sekunden dauern. Der Radius des Bereichs des Auges, welcher vermessen wird, beträgt typischerweise 3.75 mm, kann aber davon auch abweichen. Wie bereits oben ausgeführt, kann auch eine andere Anzahl an Durchläufen, Messpunkten etc. gewählt sein. Ebenso können auch andere Trajektorien als die fermat'sche eingesetzt werden.

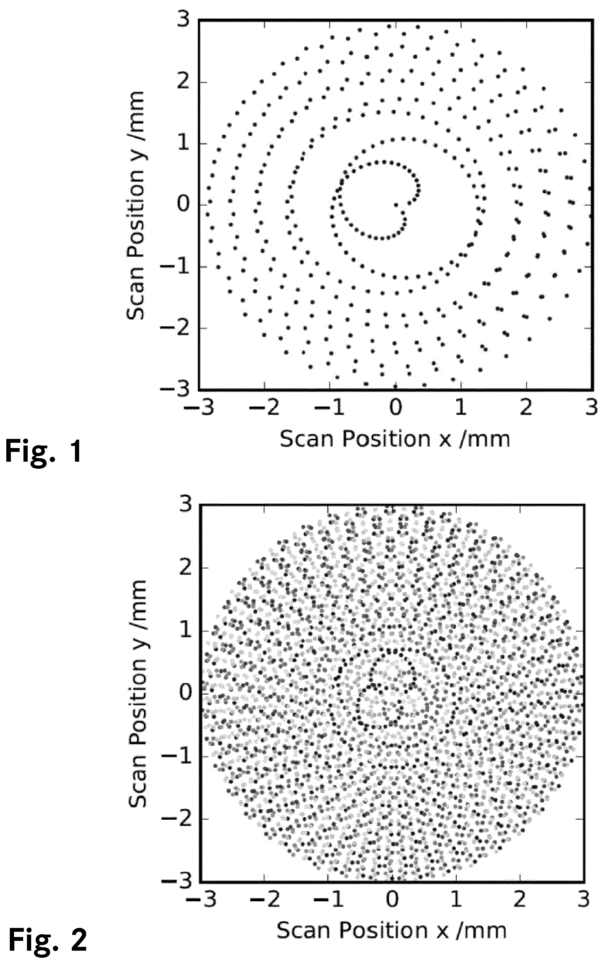

Die Figuren 1 zeigt einen ersten Zyklus der Fermatspirale gemäss obigem Beispiel in der X,Y-Ebene, wobei nur jeder zehnte Messpunkt abgebildet ist. Der Anfangs- und Endpunkt des Zyklus liegt im Apex respektive im Zentrum. Im Zentrum läuft das Ende des Zyklus in einem spitzen Winkel mit dem Anfang des Zyklus zusammen, was auf die kontinuierliche Rotation um den Winkel von $\theta_G$ = 0.375 * 2π zurück zu führen ist.

Die Figur 2 zeigt schliesslich den kompletten Scan nach acht Zyklen in der X,Y-Ebene. Dabei ist ersichtlich, dass eine sehr gute Messpunktverteilung erreicht ist.

In diesem Ausführungsbeispiel ist besonders gut ersichtlich, dass die Anforderungen an die Dynamik des OCT-Scanners relativ tief gehalten sind, da die Krümmungsradien der Trajektorie über den gesamten Durchlauf oder Zyklus verhältnismässig gross sind. Im Bereich des Apex ist die Trajektorie beinahe tangential zur Steigung der Kornea ausgerichtet. Damit wird das Verhältnis zwischen Signal und Rauschen gegenüber einem herkömmlichen Raster verbessert.

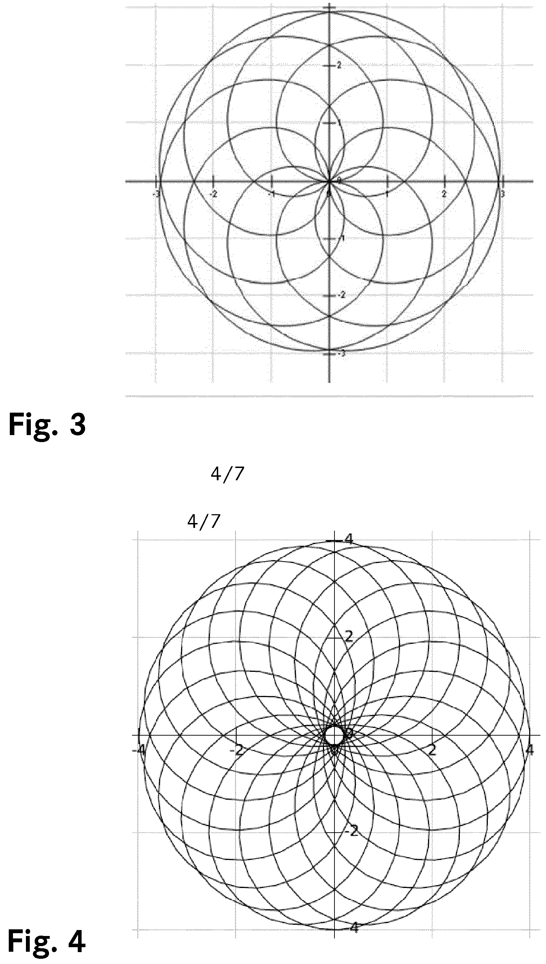

In den nachfolgenden Figuren 3 und 4 sind weitere mögliche Trajektorien der allgemeinen Form:

$$x(t) = r_0 * \sin(\omega_B t) * \cos(\omega_T t)$$

$$y(t) = r_0 * \sin(\omega_B t) * \sin(\omega_T t)$$

dargestellt. Dabei sind:

$r_0$: Radius des Umkreises des Scanmusters

$$\omega_B: \qquad \omega_B = 2\pi \frac{B}{2t_{pattern}},$$

$$\omega_T: \qquad \omega_T = 2\pi \frac{T}{t_{pattern}}$$

Für die nachfolgenden Beispiele beträgt die Messdauer $t_{pattern}$ 200 ms (Millisekunden). Dem Fachmann ist klar, dass prinzipiell eine kleinstmögliche Messdauer angestrebt ist. Diese ist jedoch einerseits vom eingesetzten Messgerät und andererseits von der Anzahl Messpunkte abhängig.

Die Anzahl Messpunkte ist vorliegend 3200, die Messfrequenz (d.h. die Rate mit welcher Messpunkte erfasst werden) beträgt f = 16 kHz. Angestrebt wird dabei ein Gleichgewicht, bei welchem die Messdauer hinreichend klein und gleichzeitig die Anzahl Messpunkte und damit bei konstanter zu vermessender Fläche die Auflösung hinreichend gross ist.

Weiterhin ist die Messfrequenz jedoch nur so gross, dass sich pro Messpunkt noch eine ausreichende Signalstärke ergibt, da diese mit zunehmender Messfrequenz abnimmt.

Die Figur 3 zeigt eine Ausführungsform einer möglichen Trajektorie in einer besonders bevorzugten Form mit B = 8 und T = 7. Am Funktionsgraphen ist gut zu erkennen, dass der Krümmungsradius jeweils vom Randbereich aus hin zum Zentrum zunimmt. Zudem liegen jeweils acht Schnittpunkte immer auf einem zum Zentrum des Umkreises konzentrischen Kreis und der Mittelpunkt wird mehrfach durchlaufen. Weiter ist in der Figur ersichtlich, dass sowohl der Randbereich als auch der zentrumsnahe Bereich hoch aufgelöst vermessen werden kann. Das Scanmuster weist 48 einfache Schnittpunkte und einen achtfachen Schnittpunkt im Zentrum auf. Insbesondere mit den Schnittpunkten ausserhalb des Zentrums kann die Augenbewegung detektiert und eliminiert werden. Die hohe Anzahl an Schnittpunkten erlaubt eine Detektion der Augenbewegung in entsprechend hoher Frequenz (Messzeit/Anzahl Schnittpunkte = mittlere Aktualisierungszeit). Die Rotation nach jedem Zyklus beträgt vorliegend $\theta_G$ = 0.4375 * 2π, so dass die Trajektorie nach 16 Zyklen wieder in der ursprünglichen Orientierung vorliegt.

Die Figur 4 zeigt schliesslich als weiteres Beispiel eine hypotrochoide Trajektorie als mögliche Ausführungsform. Die hypotrochoide Trajektorie weist die allgemeine Form:

$$x(t) = (a - b)cos(s) + c * cos\left(\left(\frac{a-b}{b}\right) * s\right);$$

$$y(t) = (a - b)sin(s) - c * sin\left(\left(\frac{a-b}{b}\right) * s\right)$$

auf.

Für die Ermittlung von Messwerten in der Augenheilkunde können die Werte derart gewählt werden, dass wiederum ein Radius von ungefähr 4 mm erreicht wird. Als Beispiel ist in der Figur 4 a=2, b=0.1 und c=2.1 gewählt. Mit dieser Parametrisierung ist im Zentrum des Umkreises ein freier Kreis zu erkennen, welcher einen Radius von ungefähr 0.2 mm aufweist. Diese Freifläche erfüllt damit das eingangs genannte 0.5 mm-Kriterium.

Die Rotation nach jedem Zyklus beträgt vorliegend $\theta_G$ = 0.56 * 2π, so dass die Trajektorie nach 25 Zyklen wieder in der ursprünglichen Orientierung vorliegt.

In einem weiteren Ausführungsbeispiel wird ein Raster aus parallelen Zeilen als Trajektorie eingesetzt, wobei das Raster nach jedem Durchlauf um zum Beispiel 10% des Zeilenabstands in der X,Y-Ebene rechtwinklig zu einer Zeilenrichtung verschoben wird. In einer weiteren Ausführungsform erfolgt jeweils eine Verschiebung um die Hälfte des letzten Zeilenabstands, womit eine kontinuierliche Verfeinerung der Messpunktverteilung erreicht werden kann. In einer weiteren Ausführungsform wird das Raster, wie eingangs erläutert, bei jedem Durchlauf um einen Winkel rotiert. In einer weiteren Ausführungsform wird schliesslich das Raster nach jedem Durchlauf sowohl Rotiert als auch verschoben.

Während in den obigen Ausführungsbeispielen jeweils die Trajektorie während des Durchlaufs kontinuierlich rotiert und/oder verschoben wird, kann die Trajektorie auch jeweils zwischen den Durchläufen rotiert und/oder verschoben werden. Dies hat jedoch in der Regel die Folge, dass zwischen den Durchläufen eine grössere Richtungsänderung durch den Messstrahl durchlaufen werden muss, womit wiederum das Messverfahren verlangsamt wird.

In jedem der obig aufgeführten Ausführungsbeispiele wird von Vorteil zur Korrektur der Bewegungsartefakten nach jedem Durchlauf oder nach jedem Sweep ein Modell des Bereichs des Auges erstellt. Anhand des Modells wird ein Symmetrievektor bestimmt, womit die Orientierung des Auges bezüglich einer Rotation um den Symmetrievektor wie auch in der X,Y-Ebene bestimmt werden kann. Mit den mehreren Symmetrievektoren wird anschliessend vorzugsweise eine Bewegungstrajektorie des Auges ermittelt. Mittels der Bewegungstrajektorie des Auges kann schliesslich jedes zuvor ermittelte Modell des Bereichs des Auges ausgerichtet werden. Die ausgerichteten Bereiche des Auges können vorteilhaft wiederum zu einem mittleren Modell des Bereichs des Auges verrechnet werden. Dem Fachmann sind beliebige Varianten zur der vorliegenden Korrektur der Bewegungsartefakte bekannt. So kann die Bewegungstrajektorie zur Korrektur der einzelnen Punkte verwendet werden etc.

Je nach eingesetztem Messgerät können die Messdauer und Anzahl Messpunkte auch kleiner respektive höher sein. Je nach Messanordnung kann es von Vorteil sein, wenn die Messdauer verkürzt wird, wobei die kleinere Auflösung in Kauf genommen wird. Anderseits kann auch auf Kosten der Messdauer die Anzahl Messpunkte erhöht werden.

Der Radius der zu vermessenden Fläche ist vorliegend zwischen 3 und 4 mm. Dieser ist jedoch ebenfalls von den spezifischen Anforderungen abhängig und kann prinzipiell beliebig gewählt werden, zum Beispiel 10 mm, 3.5 mm, 1.5 mm sowie sämtliche dazwischenliegenden und aussen liegenden Bereiche.

Die axiale Systemauflösung des Messgeräts ist vorliegend bei ungefähr 4.6 $\mu$m, aber auch diese kann höher oder niedriger sein.

Dem Fachmann ist klar, dass der Durchmesser, die Anzahl Messpunkte wie auch die Messzeit in anderen Bereichen liegen kann.

Je nach Messsystem kann die Messfrequenz von wenigen kHz bis zu einigen MHz betragen. Als erstrebenwert haben sich Messfrequenzen im Bereich von 10 bis 200 kHz erwiesen.

Schliesslich ist dem Fachmann auch klar, dass die Trajektorie nicht auf die exakte Einhaltung der durch die angegebenen mathematischen Formeln gebildeten Graphen beschränkt ist. Eine Trajektorie oder ein Scanmuster kann auch von der mathematisch exakten Form abweichen. So kann zum Beispiel die mit dem Messstrahl ermittelte Punkteschar lediglich als Interpolation näherungsweise einer solchen Funktion entsprechen.

Zusammenfassend ist festzustellen, dass erfindungsgemäss ein Verfahren zum interferometrischen Erfassen von Messpunkten eines Bereichs eines Auges geschaffen wird, welches eine besonders präzise Erfassung der Topographien des Bereichs erlaubt. Weiter wird damit eine Bewegungskorrektur des Auges in vorteilhafter Weise erreicht.

**Patentansprüche**

1. Verfahren zum interferometrischen Erfassen von Messpunkten eines Bereich eines Auges, wobei mehrere Messpunkte mit einem Messstrahl entlang einer Trajektorie erfasst werden, wobei dieselbe Trajektorie vom Messstrahl mindestens in einem ersten Durchlauf und in einem zweiten Durchlauf im Bereich durchlaufen wird, **dadurch gekennzeichnet, dass** die Trajektorie des ersten Durchlaufs zur Trajektorie des zweiten Durchlaufs um einen Winkel rotiert und/oder um eine Strecke verschoben ist, um eine homogenere Messpunktverteilung zu erreichen.

2. Verfahren nach Anspruch 1, **dadurch gekennzeichnet, dass** jede innerhalb der Trajektorie verlaufende Gerade die Trajektorie in mindestens zwei zueinander beabstandeten Punkten schneidet.

3. Verfahren nach Anspruch 1 oder 2, **dadurch gekennzeichnet, dass** ein Anfangspunkt der zweiten Trajektorie mit dem Endpunkt der ersten Trajektorie übereinstimmt.

4. Verfahren nach einem der Ansprüche 1 bis 3, **dadurch gekennzeichnet, dass** die Trajektorie den Bereich abdeckt.

5. Verfahren nach einem der Ansprüche 1 bis 4, **dadurch gekennzeichnet, dass** die Trajektorie kontinuierlich um einen Winkel rotiert und/oder um eine Strecke verschoben wird.

6. Verfahren nach einem der Ansprüche 1 bis 5, **dadurch gekennzeichnet, dass** die Trajektorie des zweiten Durchlaufs zur Trajektorie des ersten Durchlaufs ausschliesslich aufgrund einer Bewegung des Auges um einen Winkel rotiert und/oder um eine Strecke verschoben ist, womit eine homogenere Messpunktverteilung erreicht ist.

7. Verfahren nach einem der Ansprüche 1 bis 5, **dadurch gekennzeichnet, dass** die Trajektorie nach jedem Durchlauf um einen Winkel von m*360°/n, mit m, n $\geq$ 2 und m $\neq$ n rotiert ist.

8. Verfahren nach Anspruch 7, **dadurch gekennzeichnet, dass** die Trajektorie nach dem zweiten Durchlauf im Vergleich zum ersten Durchlauf um einen Winkel zwischen $360 * 0.9 * \left(\frac{3\sqrt{5}}{2}\right)$ und $360 * 1.1 * \left(\frac{3\sqrt{5}}{2}\right)$, vorzugsweise zwischen $360 * 0.95 * \left(\frac{3\sqrt{5}}{2}\right)$ und $360 * 1.05 * \left(\frac{3\sqrt{5}}{2}\right)$, besonders bevorzugt zwischen $360 * 0.99 * \left(\frac{3\sqrt{5}}{2}\right)$ und $360 * 1.01 * \left(\frac{3\sqrt{5}}{2}\right)$, insbesondere um einen Winkel von ungefähr $360 * \left(\frac{3\sqrt{5}}{2}\right)$ rotiert ist.

9. Verfahren nach einem der Ansprüche 1 bis 8, **dadurch gekennzeichnet, dass** mindestens anhand der Messpunkte des ersten Durchlaufs ein erstes Modell des Bereichs und anhand der Messpunkte des zweiten Durchlaufs ein zweites Modell des Bereichs berechnet wird.

10. Verfahren nach Anspruch 9, **dadurch gekennzeichnet, dass** anhand des mindestens ersten Modells und des zweiten Modells eine Raumkurve berechnet wird, welche die Bewegung des Auges repräsentiert.

11. Verfahren nach Anspruch 10, **dadurch gekennzeichnet, dass** für das erste Modell und das zweite Modell Symmetrievektoren bestimmt werden, anhand derer die Raumkurve berechnet wird, welche die Bewegung des Auges repräsentiert.

12. Verfahren nach Anspruch 11, **dadurch gekennzeichnet, dass** die Bewegung translatorische und rotierende Bewegungsanteile umfasst.

13. Verfahren nach einem der Ansprüche 9 bis 12, **dadurch gekennzeichnet, dass** aus den Modellen ein mittleres Modell berechnet wird.

14. Verfahren nach einem der Ansprüche 9 bis 13, **dadurch gekennzeichnet, dass** Messpunkte aufgrund des ersten Modells und des zweiten Modells, zur Reduktion von Bewegungsartefakten, zu korrigierten Messpunkten korrigiert werden.

15. Verfahren nach einem der Ansprüche 9 bis 14, **dadurch gekennzeichnet, dass** zur Korrektur der Messpunkte das mindestens erste Modell und zweite Modell interpoliert werden.

16. Verfahren nach Anspruch 15, **dadurch gekennzeichnet, dass** die korrigierten Messpunkte des ersten Durchlaufs und des zweiten Durchlaufs zu einem kumulierten Modell zusammengefasst werden.

17. Verfahren nach einem der Ansprüche 1 bis 16, **dadurch gekennzeichnet, dass** die Trajektorie in m Durchläufen durchlaufen wird, wobei m derart gewählt ist, dass ein mittlerer Messpunktabstand kleiner als eine vorherbestimmte erwartete laterale Bewegung des Auges ist.

18. Verfahren nach einem der Ansprüche 1 bis 17, **dadurch gekennzeichnet, dass** die Trajektorie die Form

   a. einer Spirale, insbesondere einer Fermatspirale;
   b. einer Hypotrochoiden;
   c. eines Rasters, insbesondere parallele Linien; oder
   d. von radial angeordneter Schleifen mit einem gemeinsamen Schnittpunkt

aufweist.

19. Vorrichtung zur Durchführung eines Verfahrens nach einem der Ansprüche 1 bis 18.

**Fig. 1**

**Fig. 2**

**Fig. 3**

4/7

**Fig. 4**

Europäisches
Patentamt

European
Patent Office

Office européen
des brevets

**EUROPÄISCHER RECHERCHENBERICHT**

Nummer der Anmeldung

EP 16 15 9900

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| E | EP 3 021 071 A1 (HAAG AG STREIT [CH]) 18. Mai 2016 (2016-05-18) * Zusammenfassung * * Absatz [0009] - Absatz [0016] * * Absatz [0086] - Absatz [0096]; Abbildungen 4-6 * * Absatz [0097] - Absatz [0098]; Abbildung 7 * | 1-5,7, 18,19 | INV. G01B9/02 G01B11/24 A61B3/10 |
| X,D | US 9 101 294 B2 (BAGHERINIA HOMAYOUN [US] ET AL) 11. August 2015 (2015-08-11) * Zusammenfassung * * Spalte 3, Zeile 63 - Spalte 5, Zeile 27 * * Spalte 6, Zeile 4 - Spalte 10, Zeile 32; Abbildungen 1-5 * * Spalte 13, Zeile 21 - Spalte 15, Zeile 48; Abbildung 9 * | 1-19 | |
| X,D | US 8 403 481 B2 (IZATT JOSEPH A [US] ET AL) 26. März 2013 (2013-03-26) * Spalte 4, Zeile 60 - Seite 6, Zeile 11 * * Spalte 17, Zeile 5 - Spalte 18, Zeile 27; Abbildungen 11-14 * | 1-5,7,8, 17-19 | RECHERCHIERTE SACHGEBIETE (IPC) G01B A61B |
| X | HONG YOUNG-JOO ET AL: "Eye motion corrected OCT imaging with Lissajous scan pattern", PROGRESS IN BIOMEDICAL OPTICS AND IMAGING, SPIE - INTERNATIONAL SOCIETY FOR OPTICAL ENGINEERING, BELLINGHAM, WA, US, Bd. 9693, 4. März 2016 (2016-03-04), Seiten 96930P-96930P, XP060064180, ISSN: 1605-7422, DOI: 10.1117/12.2212227 ISBN: 978-1-5106-0027-0 * das ganze Dokument * | 1,19 | |

-/--

Der vorliegende Recherchenbericht wurde für alle Patentansprüche erstellt

| Recherchenort | Abschlußdatum der Recherche | Prüfer |
|---|---|---|
| München | 9. September 2016 | Burkart, Johannes |

Europäisches
Patentamt

European
Patent Office

Office européen
des brevets

**EUROPÄISCHER RECHERCHENBERICHT**

Nummer der Anmeldung

EP 16 15 9900

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | OSCAR MARTÍNEZ-GRAULLERA ET AL: "2D array design based on Fermat spiral for ultrasound imaging", ULTRASONICS, Bd. 50, Nr. 2, 19. September 2009 (2009-09-19), Seiten 280-289, XP055210119, ISSN: 0041-624X, DOI: 10.1016/j.ultras.2009.09.010 * Zusammenfassung * * Abschnitte 1,2; Abbildung 1 * ----- | 7,8 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 9. September 2016 | Burkart, Johannes |

Seite 2 von 2

## ANHANG ZUM EUROPÄISCHEN RECHERCHENBERICHT
## ÜBER DIE EUROPÄISCHE PATENTANMELDUNG NR.

EP 16 15 9900

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-09-2016

| Im Recherchenbericht angeführtes Patentdokument | Datum der Veröffentlichung | Mitglied(er) der Patentfamilie | Datum der Veröffentlichung |
|---|---|---|---|
| EP 3021071 A1 | 18-05-2016 | CN 105686793 A<br>EP 3021071 A1<br>JP 2016093507 A<br>US 2016128565 A1 | 22-06-2016<br>18-05-2016<br>26-05-2016<br>12-05-2016 |
| US 9101294 B2 | 11-08-2015 | EP 2804519 A1<br>JP 2015504740 A<br>US 2013188140 A1<br>US 2016038021 A1<br>WO 2013107649 A1 | 26-11-2014<br>16-02-2015<br>25-07-2013<br>11-02-2016<br>25-07-2013 |
| US 8403481 B2 | 26-03-2013 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9101294 B2

- US 8403481 B2